# EUROPÄISCHE PATENTSCHRIFT

(11) **EP 4 450 116 B1**
(45) Veröffentlichungstag und Bekanntmachung des Hinweises auf die Patenterteilung: **23.07.2025**
(21) Anmeldenummer: 24170160.6
(22) Anmeldetag: 15.04.2024
(51) Int. Cl.: A61M 39/18, A61M 39/20, F16L 23/04, F16L 57/00, A61M 39/16

(54) **KOPPLUNGSELEMENT FÜR EINE VERBESSERTE STERILE FLANSCHVERBINDUNG**
COUPLING MEMBER FOR AN IMPROVED STERILE FLANGE CONNECTION
ÉLÉMENT DE COUPLAGE POUR RACCORDEMENT À BRIDES STÉRILE AMÉLIORÉ

(30) Priorität: 18.04.2023 AT 502862023
(43) Veröffentlichungstag der Anmeldung: 23.10.2024
(73) Patentinhaber: Zeta GmbH, 8501 Lieboch (AT)
(72) Erfinder: Kogler, Michael, 8510 Stainz (AT); Pöschl, Georg, 8102 Semriach (AT); Pittermann, Birgit, 8504 Preding (AT); Weingartshofer, Reinhard, 8561 Hitzendorf (AT)
(74) Vertreter: Schwarz & Partner Patentanwälte GmbH

(56) Entgegenhaltungen:
- EP-A1- 3 992 516
- EP-A2- 0 645 573
- FR-A1- 3 117 569
- US-A1- 2018 187 811

## Beschreibung

Die Erfindung betrifft ein Kopplungselement für eine sterile Flanschverbindung zur Verbindung von Fluidkanälen, umfassend eine fluidkontaktierende Fläche, ein erstes Öffnungsende und ein zweites Öffnungsende der fluidkontaktierenden Fläche, einen Flanschteil am ersten Öffnungsende und/ oder am zweiten Öffnungsende, eine Anschlussebene, wobei die Anschlussebene normal zu einer axialen Kopplungselementachse des Kopplungselements angeordnet ist, und das jeweilige Öffnungsende des Kopplungselements begrenzend abschließt, und eine Folie, wobei die Folie zumindest das erste Öffnungsende der fluidkontaktierenden Fläche verschließt.

Produkte, wie pharmazeutische Wirkstoffe, die biotechnologisch hergestellt werden, unterliegen strengen gesetzlichen Anforderungen, welche die Qualität der Produkte sichern sollen. Dabei sind höchst sterile Bedingungen bei der Produktion, Lagerung und Transport zu schaffen. Diese Anforderungen der sterilen Bedingungen sollen auch bei der Leitung von Flüssigkeiten zwischen Materialbehältern, Verarbeitungs- und Analysegeräten eingehalten werden, wobei besonders bei der Verbindung, bzw. Kopplung, von einem Fluidkanal mit einem anderen Fluidkanal die Gefahr besteht, unerwünschte Kontaminationen in Produkte einzubringen. Die Bereitstellung einer Verbindung von Fluidkanälen welche mehrmalig lösbar und verbindbar ist, unter Einhaltung von Sterilität ist daher von besonderer Bedeutung, da gerade beim Verbinden eine Eintrittspforte für Kontamination, aus der im allgemeinen unsterilen Umgebung, erhalten ist. Unter Kontaminationen können unerwünschte Verunreinigungen verstanden werden, einschließlich biologischer Verunreinigungen wie Viren oder winzige Organismen, z. B. Bakterien, und Umweltverunreinigungen wie Staub oder Schmutz. Diese Kontaminationen können aus einer Vielzahl von Gründen äußerst schädlich sein.

In der Regel werden nach der Verbindung von Fluidkanälen und vor dem Leiten von Produkt, ein Reinigungszyklus durch die Produktherstellungsanlage gefahren, was mehrere reinigende Mittel umfassen kann. Dabei werden Säuren und Laugen getrennt von Spülungen mit Wasser und Dampf nacheinander durch Fluidkanäle gefördert, bis Sterilität erhalten ist. Dies nimmt beträchtliche Zeit und Energie in Anspruch, was folglich zu einem weniger wirtschaftlichen Produktherstellungsverfahren führt, und damit einen wesentlichen Nachteil darstellt. Könnten die Leitungen auch bei einem Verbinden steril gehalten werden, so müssten weniger Reinigungszyklen durch die Produktherstellungsanlage gefahren werden.

Aber nicht nur das Verhindern von Eindringen von Kontaminationen in Fluidkanälen und das Reduzieren von Reinigungszyklen ist wichtig. Auch das einfache und sichere Verbinden von Fluidkanälen ist von besonderer Bedeutung, da gerade Materialbehälter regelmäßig von einer Produktionsanlage an und abgeschlossen werden, was erhebliche wirtschaftliche Konsequenzen nach sich zieht. Daher ist es wichtig, dass das Koppeln von Fluidkanälen für einen Benutzer möglichst einfach durchführbar ist, um Zeit zu sparen, und vor allem dabei auch die Sicherheit zu haben, dass keine Kontaminationen in die Kopplung eindringen konnten, während der Benutzer das Kopplungselement manipuliert und verbindet.

Sterile Kopplungselemente gemäß dem Stand der Technik umfassen im Allgemeinen im Bereich der Öffnungsenden eine abziehbare Folie. Diese Folie ist mittels eines Klebers auf die Kopplungselemente aufgeklebt, wobei diese Folie aber auch verschweißt, gepresst, oder aufgeschrumpft sein kann. Ein bekanntes System gemäß dem Stand der Technik ist in der US 8 454 059 B2 offenbart.

Das Kopplungselement dieser gegenständlichen Schrift ist eine Weiterentwicklung des sterilen Kopplungselements, welches in der EP 39 92 516 A1 offenbart wird. In dieser Patentanmeldung wird ein Kopplungselement gezeigt, dessen Öffnungsende mit einer Folie verschließbar ist.

Ein wesentlicher Nachteil bestehender Lösungen aus dem Stand der Technik, dass beim Manipulieren, bzw. Handhaben, der Kopplungselemente Gefahr besteht, dass sich die Folie unbeabsichtigt oder frühzeitig löst, und dadurch Kontaminationen in den aufwendig sterilisierten Fluidkanal und Anschlussbereich eingetragen werden. FR 3 117 569 A1 zeigt einen Konnektor, der das aseptische Verbinden von sterilen Röhrchen erleichtert.

Es ist daher die Aufgabe der Erfindung ein verbessertes Kopplungselement für eine sterile Flanschverbindung zur Verbindung von Fluidkanälen bereitzustellen, wobei die obig genannten Nachteile bekannter Kopplungselemente ausgeräumt werden sollen. Primär soll der Nachteil des frühzeitigen, unbeabsichtigten Ablösens der Folie verhindert werden.

Erfindungsgemäß wird die vorliegende Aufgabe dadurch gelöst, dass das Kopplungselement zumindest ein von dem Kopplungselement gesondertes Folienhalteelement mit einer Folienkontaktierungsfläche, an der die Folie abziehbar verbunden ist, umfasst.

Durch das zumindest eine Folienhalteelement ist der Vorteil erhalten, dass die Folie zusätzlich oder ausschließlich zu dem gesonderten Folienhalteelement verbindbar ist, wobei eine zuverlässigere Verbindung der Folie mit dem Kopplungselement durch das Folienhalteelement erwirkt wird. Hierdurch wird effektiv erreicht, dass die Folie bei einem Manipulieren des Kopplungselements durch einen Benutzer nicht unbeabsichtigt gelöst wird, da die Haftung der Folie an das Kopplungselement nicht ausreichend ist. Dadurch ist ein wesentlich verbesserter Schutz vor Eindringen von Kontaminationen erhalten. Besonders kritisch ist ein teilweises Ablösen der Folie, da dies leicht von dem Benutzer übersehen werden kann, da die Folie den Anschein erweckt, dass die Folie noch vollständig mit dem Folienhalteelement verbunden sei.

Unter dem Begriff Flanschteil kann erstens ein mit dem Kopplungselement verbundenes Teil verstanden werden. Dabei kann der Flanschteil geklebt oder geschweißt vorliegen. Zweitens ist darunter ebenso die monolithische Ausführungsform miteingeschlossen. Der Flanschteil kann auch als loser Flanschteil vorliegen, wobei dann eine Aufdickung am jeweiligen Öffnungsende erforderlich ist, um eine Verbindung zu ermöglichen.

Vorzugsweise ist zumindest ein das erste Öffnungsende umlaufendes Dichtungselement im Bereich der Anschlussebene ausgebildet. Hierdurch wird der Vorteil erhalten, dass beim Zusammenfügen zu einer Flanschverbindung eine zusätzliche Barriere gegen das Eindringen von Kontaminationen in die Verbindung der Fluidkanäle bereitgestellt ist.

Unter Bereich der Anschlussebene ist verstanden, dass die Stärke des Dichtungselements die Anschlussebene schneidet, oder zumindest berührt. Nur so kann eine dichte und sterile Verbindung bereitgestellt werden, wenn das Kopplungselement gekoppelt wird. Beim Abziehen der Folie, während des Koppelns des Kopplungselements könnte außerdem ein Spalt entstehen, wobei dieser Spalt von dem Dichtungselement unmittelbar verschlossen wird, um so eine durchgehende Sterilität bei der Verbindung von Fluidkanälen zu erhalten. Im Allgemeinen wird beim Koppeln das Kopplungselement gegen eine andere Anschlussebene leicht angedrückt, sodass die Folie weiterhin von einem Benutzer abziehbar ist, ohne eine Eintrittspforte für Kontaminationen auszubilden.

Das zumindest eine Dichtungselement ist vorzugsweise in zumindest einer Dichtungselementvertiefung ausgebildet, wobei das Dichtungselement über die Anschlussebene hinausgehend ist und die Dichtungselementvertiefung in den Flanschteil eingearbeitet ist. Vorteilhaft dabei ist, dass das Dichtungselement durch einen Formschluss im Kopplungselement gehalten wird, ohne dass eine andere Art der Befestigung bereitgestellt werden muss. Die Dichtungselementvertiefung kann vorzugsweise mit der Form des Dichtungselements zusammenfallen. Dabei kann für ein ringförmiges O-Ring Dichtungselement, eine im Querschnitt kreisrunde gefräste Dichtungselementvertiefung bereitgestellt sein, wobei auch andere Formen der Dichtungselementvertiefungen bereitgestellt sein können. Auch ein anderer Typ von Dichtungselement kann vorliegen, wie beispielsweise Profildichtungen oder Dichtmassen.

Vorzugweise ist auch das zumindest eine Folienhalteelement im Bereich der Anschlussebene ausgebildet. Dadurch ist der Vorteil erhalten, dass die Folie mit einer geringen Fläche ausgebildet werden kann, wodurch das Auftreten von möglichen Fehlern in der Folie, durch welche Kontaminationen eindringen können, reduziert werden kann. Ist beispielsweise das Folienhalteelement nicht im Bereich der Anschlussebene bereitgestellt, so ist zwar ein größerer Teil des Kopplungselements steril verpackt, allerdings ist auch ein größerer Teil der Folie möglichen Beschädigungen, oder Fehlern, die in einer Herstellung der Folie auftreten können, ausgesetzt.

Gemäß einer Ausführungsform ist das zumindest eine Folienhalteelement in zumindest einer Folienhalteelementvertiefung ausgebildet, wobei die Folienkontaktierungsfläche des Folienhalteelements mit der Anschlussebene abschließt, wobei die Folienhalteelementvertiefung in den Flanschteil eingearbeitet ist. Durch das Abschließen mit der Anschlussebene liegen keine Kanten vor, an welchen die Folie beim Manipulieren des Kopplungselement beschädigt werden kann, da die Folie flach, ohne Ausbildung von Kanten an der Anschlussebene anliegt. Außerdem ist so der Vorteil erhalten, dass bei einem Koppeln des Kopplungselementes keine Kante, bzw. Ecke der Folienhalteelementvertiefung in punktuellen Kontakt treten können, wodurch die Widerstandsfähigkeit des Kopplungselements verbessert werden kann, da die Folienhalteelementvertiefung nicht ausgeschlagen werden kann. Bei einem Ausschlagen könnte Material, aus welchem das Kopplungselement hergestellt ist, in ein Produkt eingebracht werden, was wiederum eine kritische Kontamination darstellt.

Gemäß einer weiteren Ausführungsform, in der ein Dichtungselement und ein Folienhalteelement im Bereich der Anschlussebene ausgebildet ist, ist ein Umfang des Folienhalteelements größer als ein Umfang des Dichtungselements. Dadurch ist der Vorteil erhalten, dass das Dichtungselement von der Folie überdeckt wird, wodurch ein besonders steriles Kopplungselement erhalten ist, da Fluid im Betriebszustand vom Dichtungselemente zurückgehalten wird, und so nicht in Kontakt mit dem Flanschteil außerhalb der Dichtelemente kommen kann, wodurch die Fläche von möglicher Kontamination reduziert wird. Außerdem kommt das Dichtungselement zuerst in Kontakt mit einer anderen Anschlussfläche bei einem Koppeln des Kopplungselements. Dabei drückt das Dichtungselement die Folie beim Koppeln gegen eine andere Anschlussebene, wodurch die Verbindung schon beim Abziehen der Folie eine gewisse Dichtheit und damit Schutz gegenüber Eindringen von Kontaminationen aufweist.

Vorzugsweise ist das Folienhaltelement aus Kunststoff, bevorzugt aus Polyethylen (PE), ausgebildet und/oder das Kopplungselement ist aus Metall ausgebildet, bevorzugt aus Stahl, besonders aus poliertem Stahl. Folien, besonders ausgeführt aus Verbundmaterial, haften besonders gut an dem Werkstoff Polyethylen, wobei Polyethylen durch seine Eigenschaften besonders gut für sterile Kopplungselemente geeignet ist, da dieser Werkstoff eine hohe Zähigkeit, Nass- und Reißfestigkeit, mehrfache Verwendbarkeit und leichte Verarbeitbarkeit aufweist. Eine besonders gute Verbindung von Folienhalteelement zu Folie ist besonders dann wichtig, wenn das Kopplungselement aus Metall ausgebildet ist, da die Folie im Allgemeinen besonders schlecht an Metall haftet. Dieser Umstand ist verstärkt, wenn das Kopplungselement aus poliertem Stahl ausgebildet ist, da so fast keine Haftung der Folie zur Metalloberfläche besteht. Kopplungselemente aus poliertem Stahl sind besonders bevorzugt, da diese oft wiederverwendbar sind und da diese eine große mechanisches Festigkeit aufweisen, im Vergleich zu Kunststoffen und anderen Materialien. Außerdem ist durch die polierte, glatte Oberfläche ein Ansammeln von Kontaminationen auf der Oberfläche und der fluidkontaktierenden Fläche zusätzlich verschlechtert. Folglich ist das erfindungsgemäße Folienhalteelement bei dem Kopplungselement aus poliertem Stahl besonders vorteilhaft.

Es soll auch erwähnt werden, dass das Folienhaltelement aus pharmakonformen und anderen Kunststoffen bereitgestellt werden kann. Darunter sollen folgende Kunststoffe beispielweise genannt sein: PE 1000 REIN, PPSU, PES, PSU, PVDF, PET, POM-H, POM-C, PA 6 G 210, PA 66, PA 6, PP, PE-UHMW, PE 500, PE 300, und PE 100.

Gemäß einer Ausführungsform ist zumindest ein Durchgangsloch im Flanschteil ausgebildet, wobei das Folienhaltelement durch das Durchgangsloch erreichbar ist. Durch dieses Durchgangsloch ist der Vorteil erhalten, dass das Folienhalteelement aus dem Kopplungselemente herausgedrückt werden kann. Bei Abnutzungserscheinungen kann so besonders einfach ein neues Folienhalteelement gegen ein Abgenutztes ausgetauscht werden. Ein einfaches Herausnehmen des Folienhalteelements ist auch dann vorteilhaft, wenn das Kopplungselement besonders gründlich sterilisiert werden soll. Da in einem Spaltbereich zwischen eingebrachtem Folienhalteelement und Kopplungselement ein Sterilisierungsmittel nur schwer vordringen kann. Kann jedoch das Folienhalteelement einfach aus dem Kopplungselement entnommen werden, können beide Bauteile ohne Ausbildung eines Spaltbereichs zuverlässig gereinigt werden.

Das Durchgangsloch kann auch beim Ausbilden des Folienhalteelements einen wesentlichen Vorteil bereitstellen, da bei einem Einspritzen des Folienhalteelements durch das Durchgangsloch ein Entweichweg für Luft vorliegt, wodurch ein Einschluss von Luft effektiv unterbunden werden kann. Einschlüsse von Luft können Einbuchtungen in der Oberfläche des Folienhalteelements verursachen, an denen eine Folie nicht haften kann. Auch ist durch Einschlüsse die mechanische Festigkeit des Folienhalteelements beeinträchtigt, was im Betrieb zu Versagen führen kann.

Auch kann durch das Durchgangsloch direkt eingespritzt werden. Liegt dabei das Kopplungselement mit seiner Anschlussebene gegen eine Arbeitsfläche an, kann so ohne zusätzliche Werkzeugform das Folienhaltelement durch direktes Einspritzen in das Durchgangsloch ausgebildet werden.

Gemäß einer weiteren Ausführungsform bildet die Folienhalteelementvertiefung Widerhaken aus. Die Widerhaken begünstigen das Halten des Folienhalteelements in seiner Folienhalteelementvertiefung, da die Widerhaken einen effektiven Formschluss zum Kopplungselement bereitstellen.

Vorzugsweise weist das Folienhalteelement zumindest eine umlaufende Nut auf, wobei die Nut bevorzugt in der Folienkontaktierungsfläche und/oder in einer der Folienkontaktierungsfläche angrenzenden Fläche und/oder in einer der Folienkontaktierungsfläche abgewandten Fläche V-förmig ausgebildet sein kann.

In jedem Fall hat die umlaufende Nut den Vorteil, dass durch Temperaturänderungen bedingte Spannungen durch die Nut abgebaut werden können. Außerdem ist ein erleichterter Einbau des Folienhalteelementes in die Folienhalteelementvertiefung erhalten, da die umlaufende Nut eine höhere Verformbarkeit bereitstellt.

Ist die Nut in der Folienkontaktierungsfläche ausgebildet, kann über die Größe der Nut effektiv bestimmt werden, wie viel Kraft ein Benutzer aufwenden muss, um die Folie von der Folienkontaktierungsfläche abzuziehen. Auf der einen Seite muss das Folienhalteelement eine bestimmte Größe haben, damit dieses in der Folienhalteelementvertiefung festgehalten werden kann, ohne bei einem Abziehen der Folie zu brechen, oder mit der Folie zusammen abgezogen zu werden. Auf der anderen Seite darf die Fläche, an der die Folie verbunden ist, nicht zu groß sein, sodass diese nicht lösbar bzw. unabziehbar ist, da deren Kontaktfläche zu groß ist. Eine Nut in der Folienkontaktierungsfläche berücksichtig beide Kriterien, und kann entsprechend dimensioniert werden.

Ist die Nut V-förmig ausgeformt, so ist der weitere Vorteil erhalten, dass ein Messer zum Trennen der Folie durch einen Benutzer in dieser Nut geführt werden kann, ohne Gefahr zu laufen, die Anschlussebene des Kopplungselements zu zerkratzen. Das ist besonders dann vorteilhaft, wenn die Folie nicht in geeigneter Größe zum Verschließen der Öffnungsenden bereitgestellt ist, und am Kopplungselement selbst zugschnitten werden muss. Das ist vorteilhaft, da bei einem Überstehen der Folie über das Folienhaltelement bei einem Manipulieren des Kopplungselements Kontaminationen in den nicht verbundenen Teil der Folie eingetragen werden können. Bei einem Abziehen der Folie könnte durch Abstreifen dieser kontaminierten nicht verbundenen Teile, bzw. der unsterilen Seiten, über die Dichtungselemente oder durch Berühren der fluidkontaktierenden Fläche, Kontaminationen eingebracht werden, was folglich eine weniger sterile Verbindung nach sich zieht.

Ein weiterer Vorteil ist, dass wenn die Folienhalteelemente nicht direkt in die Folienhaltevertiefungen eingespritzt werden, sondern, extern gefertigt werden, diese bedingt durch die V-förmige Nut, leichter verformt werden können, um so in die Folienhaltevertiefung eingepresst zu werden. Die Seitenwände wirken entsprechend wie Federn, die das Halteelement durch Drücken gegen die Seitenwand der Folienhaltevertiefung festhalten.

Gemäß einer Ausführungsform ist im Bereich des ersten Öffnungsendes eine kegelstumpfförmige Aufweitung ausgebildet. Das bringt den Vorteil, da so Material vorgesehen ist, welches den Verlust der mechanischen Festigkeit ausgleicht, der durch das Bereitstellen des Folienhalteelements erhalten ist. Besonders vorteilig ist die kegelstumpfförmige Aufweitung, wenn die Dichtungselementvertiefungen und Folienhaltevertiefungen ausgebildet sind, da diese eine erhebliche Reduzierung der Festigkeit des Kopplungselements mit sich bringen. Auf gekoppelte Kopplungselemente wirken große Momente durch das Gewicht von Schlauchleitungen, weswegen das Bereitstellen von mechanischer Festigkeit essenziell ist. Ein weiter Vorteil der kegelstumpfförmigen Aufweitungen ist, dass wenn die Folie zum Folienhalteelement verbunden ist, und wenn das Folienhalteelement nicht im Bereich der Anschlussebene angeordnet ist, die Kegelstumpfform dem Abspannwinkel der Folie folgen kann. Dadurch liegen keine Bereiche am Kopplungselement vor, die von der Folie überspannt werden. Dadurch ist die Folie besser vor externem Einwirken geschützt, da die kegelstumpfförmige Aufweitung direkt hinter der Folie liegt.

Vorzugweise können in einer Flanschverbindung zwei erfindungsgemäße Kopplungselemente und ein Verbindungselement zum lösbaren Verbinden der zwei Kopplungselemente bereitgestellt sein. Dadurch ist der Vorteil erhalten, dass eine besonders sterile Verbindung von Fluidkanälen bereitgestellt werden kann, da das Zusammenwirken von zwei erfindungsgemäßen Kopplungselementen mit deren Folienhalteelementen höchst effektiv das Einbringen von Kontaminationen, durch das verbesserte Anhaften der Folien an den Folienhalteelementen, verhindert.

Das erfindungsgemäße Kopplungselement kann wie folgt beschrieben zu einer sterilen Flanschverbindung gekoppelt werden. Dabei wird zuerst eine Folie mit dem Folienhalteelement verbunden. Als nächstes wird die Folie unter Druck und hoher Temperatur versiegelt. Danach wird mit Dampf die fluidkontaktierende Fläche des Kopplungselements sterilisiert. In weiterer Folge wird das erste Öffnungsende mit dem Öffnungsende eines weiteren Kopplungselements im Wesentlichen deckungsgleich angeordnet. Danach werden die Kopplungselemente zumindest abschnittsweise im Bereich der Öffnungsenden durch ein Verbindungselement aufgenommen. In weiterer Folge werden die, die Öffnungsenden verschließenden, und von dem jeweiligen Öffnungsende abziehbaren Folien durch eine Ausnehmung in dem Verbindungselement durchgeführt. Anschließend wird die sterile Fluidverbindung durch gleichzeitiges Abziehen der Folien durch die Ausnehmung des Verbindungselements hindurch hergestellt. Im zuletzt genannten Schritt, zur Herstellung der sterilen Fluidverbindung, werden die Folien durch die Ausnehmung in dem Verbindungselement hindurch von den Öffnungsenden abgezogen. Durch das Verbindungselement wird der Vorteil erreicht, dass die Kopplungselemente aufgenommen sind, bevor die Folien durch die Ausnehmung in dem Verbindungselement abgezogen werden. Hierdurch wird gewährleistet, dass eine sichere sterile, und mechanisch stabile Verbindung der Fluidkanäle vorliegt, bevor die Folien entfernt werden. Dies wird insbesondere auch dadurch erreicht, dass hierbei die unsterilen Seiten der Folien, die an den Öffnungsenden anliegen, die sterile, beziehungsweise keimarme, fluidkontaktierende Flächen nicht berühren. Hierdurch wird effektiv verhindert, dass Fremdkörper und/oder Keime in die Fluidverbindung im Zuge des Abziehens der Folien gelangen, wodurch die Sterilität der Fluidverbindung sichergestellt wird. Zusätzlich wird die Fluidverbindung bereits vor dem Abziehen der Folien mechanisch stabilisiert, und auch gegen große mechanische Belastungen gesichert, wodurch die Sicherheit der Fluidverbindung zusätzlich erhöht wird. Durch die getrennte Ausführung des Verbindungselements von den Kopplungselementen kann die Fluidverbindung durch einfaches Abnehmen des Verbindungselements wieder getrennt werden. Nach erfolgter Reinigung, Sterilisierung und Anbringen neuer Folien an das Folienhalteelement steht das System für eine erneute Benutzung bereit. Nutzten sich die Folienhalteelemente mit mehrmaliger Verwendung ab, so können diese einfach ausgetauscht werden, ohne in die Substanz der Kopplungselemente selbst eingreifen zu müssen. Hierdurch wird die Wirtschaftlichkeit erhöht, und die Umweltverträglichkeit verbessert, wobei eine immer sicher verbundene Folge an Kopplungselementen bereitgestellt ist.

Vorteilhafte und nicht einschränkende Ausführungsformen der Erfindung werden nachfolgend anhand der Figuren näher erläutert.
Fig. 1 zeigt eine Flanschverbindung umfassend zwei Kopplungselemente, die an ihren jeweiligen ersten Anschlussebenen gekoppelt sind, wobei an einer zweiten Anschlussebene des rechten Kopplungselements ein Deckel über ein weiteres Verbindungsmittel gekoppelt ist.
Fig. 2A zeigt das Kopplungselement bzw. das andere Kopplungselement aus Fig. 1 aus der Flanschverbindung herausgelöst, isoliert, in einer ersten isometrischen Ansicht, mit einer Folie, wobei die Folie das erste Öffnungsende der fluidkontaktierenden Fläche verschließt.
Fig. 2B zeigt das Kopplungselement aus Fig. 2A ohne Folie, in einer zweiten isometrischen Ansicht, wobei ein erstes Öffnungsende und dessen Anschlussebene zu sehen sind.
Fig. 2C zeigt das Kopplungselement aus Fig. 2B, in einer dritten isometrischen Ansicht, wobei das zweite Öffnungsende zu sehen ist.
Fig. 3A zeigt das Kopplungselement aus Fig. 2A bis 2C in Schnittansicht.
Fig. 3B zeigt die Schnittansicht des Kopplungselements aus Fig. 2A, wobei im Detail die Folienhalteelementvertiefung und die Dichtungselementvertiefung als Ausschnitt vergrößert darstellt werden.
Fig. 4A zeigt das Folienhalteelement aus Fig. 1 isoliert in Schnittansicht.
Fig. 4B zeigt das Folienhalteelement aus Fig. 4A in einer ersten isometrischen Ansicht.
Fig. 4C zeigt das Folienhalteelement aus Fig. 4A in einer zweiten isometrischen Ansicht.
Fig. 5 zeigt die Flanschverbindung aus Fig. 1 ungeschnitten in einer isometrischen Ansicht.

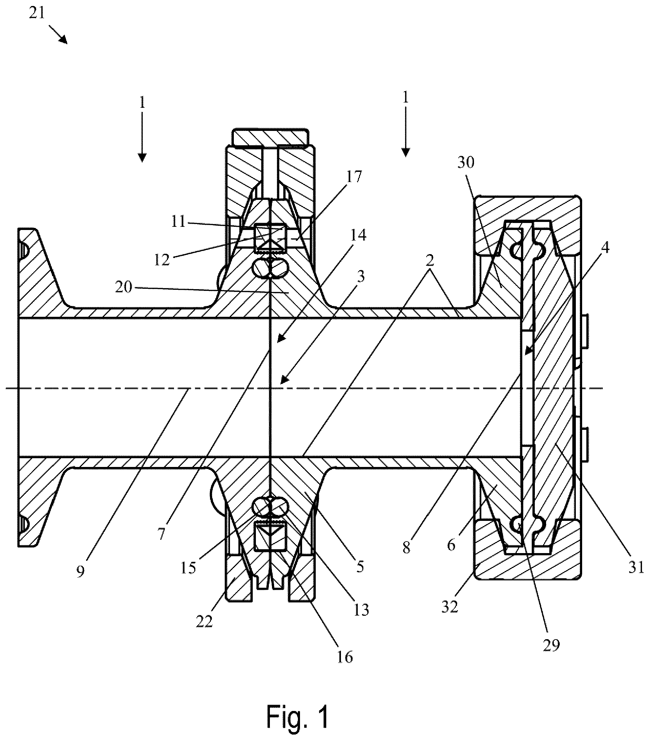

Fig. 1 zeigt eine lösbare Flanschverbindung umfassend ein erstes erfindungsgemäßes Kopplungselement 1 und ein weiteres erfindungsgemäßes Kopplungselement 1.

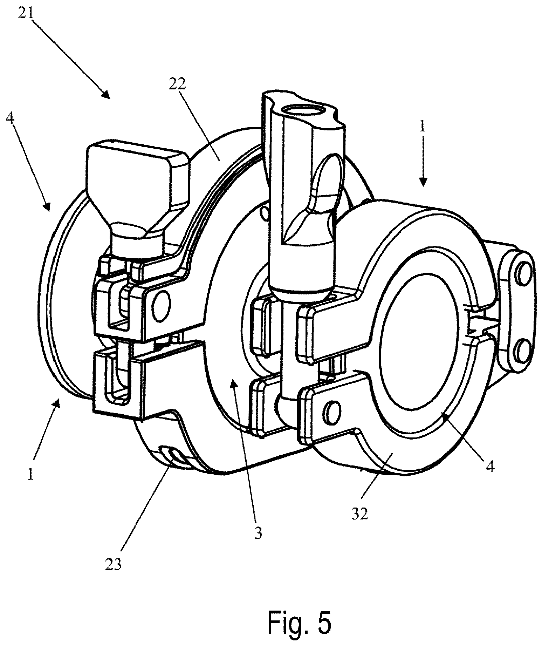

Dabei sei angemerkt, dass die folgenden Erläuterungen auf das rechte der beiden Kopplungselemente bezogen sind, wobei einer Fachperson aber klar ist, dass die Erläuterungen auf das weitere linke Kopplungselement übertragbar sind. Beide Kopplungselemente teilen sich das Bezugszeichen 1, da diese in der dargestellten Flanschverbindung 21 in Fig. 1 und Fig. 5 baugleich ausgeführt sind. Dabei ist einer Fachperson klar, dass das andere Kopplungselement nicht zwangsweise baugleich ausgebildet sein muss.

Die zwei Kopplungselemente 1 sind, an ihren jeweiligen ersten Anschlussebenen 7, in Kontakt gebracht und gekoppelt, wobei die lösbare Kopplung über ein Verbindungelement 22 bereitgestellt wird. An das rechte Kopplungselement 1 ist an eine zweite Anschlussebene 8 ein Deckel 31 über ein weiteres Verbindungselement 32 lösbar gekoppelt.

Das Kopplungselement 1 für die, in Fig. 1 gezeigte, sterile Flanschverbindung 21 zur Verbindung von Fluidkanälen umfasst eine fluidkontaktierende Fläche 2, ein erstes Öffnungsende 3 und ein zweites Öffnungsende 4 der fluidkontaktierenden Fläche 2, einen Flanschteil 5 am ersten Öffnungsende 3 und einen Flanschteil 6 am zweiten Öffnungsende 4. Die Anschlussebene 7 steht normal zu einer axialen Kopplungselementachse 9 des Kopplungselements 1, und das erste Öffnungsende 3 schließt das Kopplungselement 1 begrenzend ab. Unter begrenzend abschließen ist in dieser Ausführungsform zu verstehen, dass kein Teil des Kopplungselements 1 über die Anschlussebene 7 hervorsteht.

Das Kopplungselement 1 weist zumindest ein von dem Kopplungselement 1 gesondertes Folienhalteelement 11 mit einer Folienkontaktierungsfläche 12 auf, an der eine Folie 10 abziehbar verbunden ist. Die Folie 10 ist in Fig. 1 nicht dargestellt, da der Kopplungszustand nach Abziehen der Folien 10 und Schließen des Verbindungselements 22 gezeigt ist. Mit anderen Worten kommunizieren die beiden Kopplungselemente 1 bereits miteinander.

Im Kopplungselement 1 ist ein das erste Öffnungsende 3 umlaufendes Dichtungselement 13 im Bereich 14 der Anschlussebene 7 ausgebildet. Das Dichtungselement 13 ist in einer Dichtungselementvertiefung 15 ausgebildet, wobei das Dichtungselement 13 über die Anschlussebene 7 hinausgehend ist und die Dichtungselementvertiefung 15 in den Flanschteile 5 eingearbeitet ist. Das Kopplungselement 1 weist außerdem eine zweite Dichtungselementvertiefung 29 in einem Flanschteil 6 am zweiten Öffnungsende 4 auf, wobei kein Dichtungselement darin dargestellt wird, da die Flanschverbindung 21 herausgelöst aus ihrer tatsächlichen Betriebslage im allgemeinen Kontext einer Produktherstellung dargestellt ist. Im Allgemeinen ist das erste Kopplungselement 1 an einen Behälter mit einer Produktkomponente über das Flanschteil 6 am zweiten Öffnungsende 4 gekoppelt. Das andere Kopplungselement 1 ist im Allgemeinen über eine flexible Schlauchleitung an den Rest der Produktherstellungsanlage gekoppelt.

Das Folienhalteelement 11 ist wie das Dichtungselement 13 im Bereich 14 der ersten Anschlussebene 7 ausgebildet, wobei das Folienhalteelement 11 in einer Folienhalteelementvertiefung 16 bereitgestellt ist, wobei die Folienkontaktierungsfläche 12 des Folienhalteelements 11 mit der ersten Anschlussebene 7 abschließt. Die Folienhalteelementvertiefung 16 ist in den Flanschteil 5 eingearbeitet.

Ein Umfang des Folienhalteelements 11 ist größer als ein Umfang des Dichtungselements 13. Unter Umfang kann im Kontext eines Dichtungselements 13 sein Innendurchmesser verstanden werden, wobei der Begriff auf nicht runde Dichtungselemente zu erweitern ist. Das Dichtungselement 13 kann beispielsweise auch als Flachdichtungen ausgebildet sein, wobei als Umfang dessen Flächenbegrenzende verstanden wird.

Das Kopplungselement 1 weist ein Durchgangsloch 17 auf, welches im Flanschteil 5 ausgebildet ist, wobei das Folienhaltelement 11 durch das Durchgangsloch 17 erreichbar ist. Unter erreichbar kann verstanden werden, dass ein Benutzer mit einem Werkzeug das Folienhalteelement 11 durch das Durchgangsloch 17 hindurch berühren kann.

Im Bereich 14 des ersten Öffnungsendes 3 ist eine kegelstumpfförmige Aufweitung 20 ausgebildet, wobei in dieser Ausführungsform auch eine zweite kegelstumpfförmige Aufweitung 30 im Bereich des zweiten Öffnungsendes 4 ausgebildet ist.

Vorzugsweise ist die Flanschverbindung 21 für einen Druckbereich von 0 bis 10 bar Druck in der Fluidverbindung ausgelegt. Gemäß der erfindungsgemäßen Ausführungsform können auch das Kopplungselement 1 und das andere Kopplungselement 1 im Gebrauch mit einem Schlauch verbunden und desinfiziert werden.

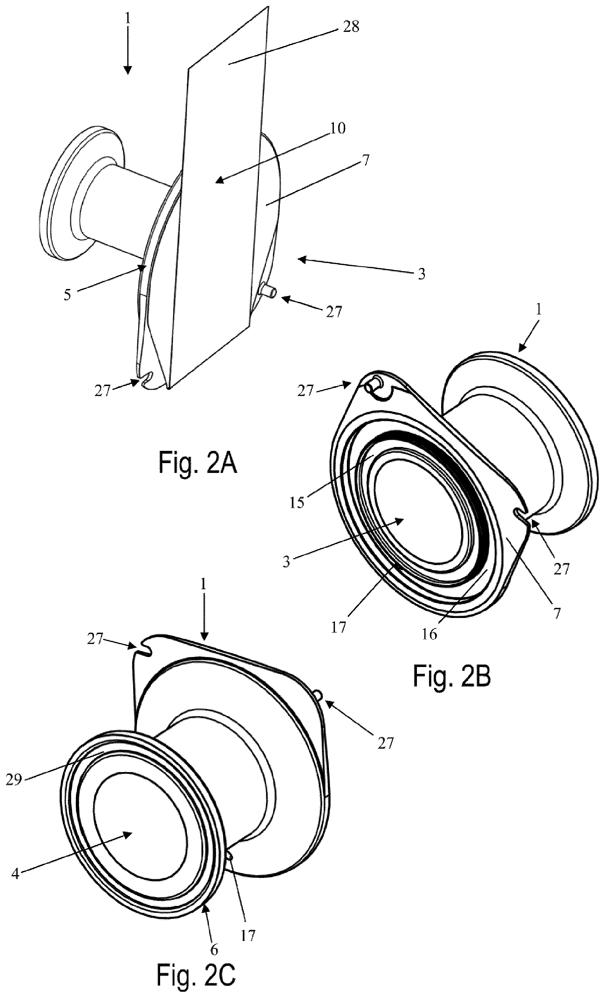

Fig. 2A zeigt das Kopplungselement 1 bzw. das andere Kopplungselement 1 aus Fig. 1 aus der Flanschverbindung 21 herausgelöst, isoliert, in einer ersten isometrischen Ansicht, mit einer Folie 10, wobei die Folie 10 im Gegensatz zu Fig. 1 dargestellt wird. Die Folie 10 verschließt das erste Öffnungsende 3 der fluidkontaktierenden Fläche 2. Die Folie 10 ist an einer ersten Anschlussebene 7, welche normal zu einer axialen Kopplungselementachse 9 des Kopplungselements 1 angeordnet ist, verbunden. Dabei ragt eine greifbare Fläche 28 der Folie 10 über das Flanschteil 5 hinaus, sodass ein Benutzer diese greifbare Fläche 28 der Folie 10 greifen und abziehen kann.

Die Folie 10 ist vorzugsweise als Verbundfolie aus handelsüblichen, pharmagerechten und/oder lebensmittelechten Materialien ausgebildet, die in einer Stanzmaschine vorgefertigt wird. Auf diese Weise kann das Kopplungselement 1 in einem großen Druck- und Temperaturbereich eingesetzt werden. Die vorgefertigte Folie 10 wird vorzugsweise mit Hilfe von Druck und/oder Temperatur auf das Folienhalteelement 11 am Öffnungsende 3 aufgebracht. Durch die Hitze schmilzt das Material der Folie 10 und kann nach dem Abkühlen rückstandsfrei abgezogen werden.

Fig. 2B zeigt das Kopplungselement 1 aus Fig. 2A ohne Folie 10, bzw. aus Fig. 1, in einer zweiten isometrischen Ansicht, wobei das erste Öffnungsende 3 und dessen Anschlussebene 7 zu sehen sind. Die Folienhalteelementvertiefung 16, ist ohne Folienhalteelement 11 dargestellt. Auch ist die Dichtungselementvertiefung 15 ohne Dichtungselement 13 dargestellt.

Fig. 2C zeigt das Kopplungselement 1 aus Fig. 2B, in einer dritten isometrischen Ansicht, wobei das zweite Öffnungsende 4 zu sehen ist. Die zweite Dichtungselementvertiefung 29 im zweiten Flanschteil 6 am zweiten Öffnungsende 4 ist wiederum ohne Dichtungselement dargestellt.

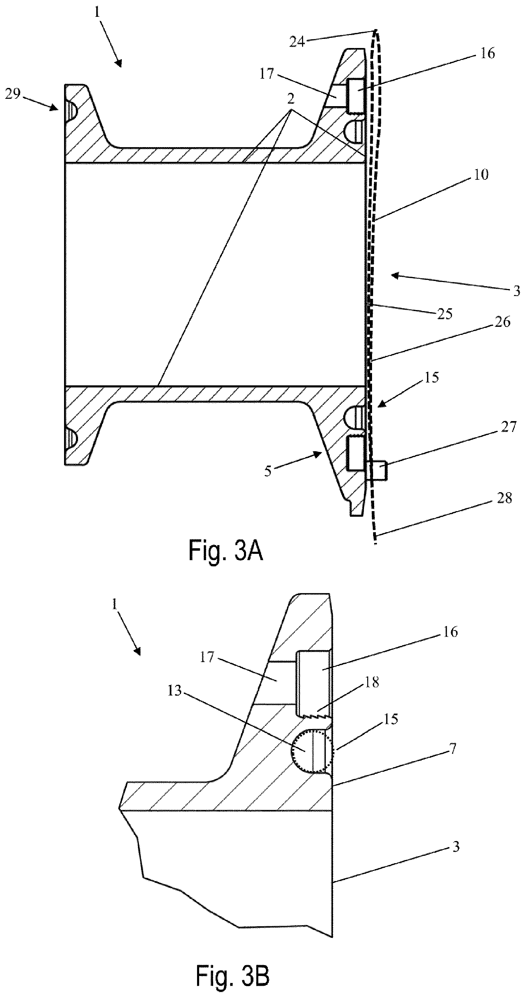

Fig. 3A zeigt eines der beiden Kopplungselemente 1 aus Fig. 1 bis Fig. 2C in einer Schnittansicht. Dabei sind in den Dichtungselementvertiefungen 15, 29 und in der Folienhaltelementvertiefung 16 keine Dichtungselemente 13 und kein Folienhalteelement 11 dargestellt. Allerdings ist eine Folie 10, welche das erste Öffnungsende 3 verschließt, strichliert dargestellt. Dabei ist die Folie 10 einmal entlang ihrer Breitseite gefaltet, sodass eine greifbare Fläche 28 nach unten hinweg über das Flanschteil 5 hinaussteht. Dadurch ist der Vorteil erhalten, dass beim Koppeln in einer Flanschverbindung 21 (wie es in Fig. 1 dargestellt ist), die zwei in kontaktstehenden Folien 10 voneinander abrollbar sind, und das Ablösen der Folie 10 durch das Scherren im Umlenkbereich 24, bzw. Faltlinie, beim Abrollen vom Folienhalteelement 11 besonders leicht erfolgen kann. Außerdem werden so zuverlässig mögliche Kontaminationen, welche sich auf einer unsterilen Seite 26 der Folie 10 angesammelt haben, von der fluidkontaktierenden Fläche 2 weggetragen, ohne Gefahr, dass die unsterile Seite 26 der Folie 10 die sterilisierte fluidkontaktierende Fläche 2 berührt und Kontaminationen einbringt. Angemerkt soll sein, dass unter unsteriler Seite 26 der Folie 10 der gesamte Teil verstanden wird, der der Umwelt ausgesetzt ist. Die sterile Seite 25 der Folie 10 ist demnach der Teil der Folie 10, der mit der fluidkontaktierenden Fläche 2 kommuniziert und im Zuge einer Sterilisation des Kopplungselements 1 besonders steril bereitgestellt wird.

Fig. 3B zeigt in Schnittansicht das Kopplungselements 1 aus Fig. 2A, wobei im Detail die Folienhalteelementvertiefung 16 und die Dichtungselementvertiefung 15 als Ausschnitt vergrößert darstellt werden. In Fig. 3B ist besonders klar zu erkennen, dass die Folienhalteelementvertiefungen 16 Widerhaken 18 ausbilden. Des Weiteren ist auch die Dichtungselementvertiefung 15 besser erkennbar. In der Dichtungselementvertiefung 15 ist schematisch, mit einer gepunkteten Linie, ein Dichtungselement 13 vom Typ O-Ring dargestellt, womit verdeutlicht sein soll, dass das Dichtungselement 13 über die Anschlusseben 7 minimal hinausgehend ist. Es soll erwähnt sein, dass die Widerhaken 18 unterschiedlichst ausgebildet werden könne. Beispielweise kann lediglich ein einzelner zahnförmiger Vorsprung vorgesehen werden, um das Folienhalteelement 12 in der Folienhalteelementvertiefung 16 zu halten. Aber auch mehrere Vorsprünge können ausgebildet sein, um als Widerhaken 18 zu fungieren. Es soll ebenso erwähnt sein, dass die Widerhaken 18 an einer beliebigen Fläche der Folienhalteelementvertiefung 16 vorgesehen sein können. Ebenso müssen die Widerhaken 18 nicht die gesamte Folienhalteelementvertiefung 16 umlaufen, sondern können lediglich punktuell vorgesehen werden. Beispielsweise an drei Stellen in der Folienhalteelementvertiefung 16, wobei diese Stellen 120° Grad voneinander beabstandet sein können.

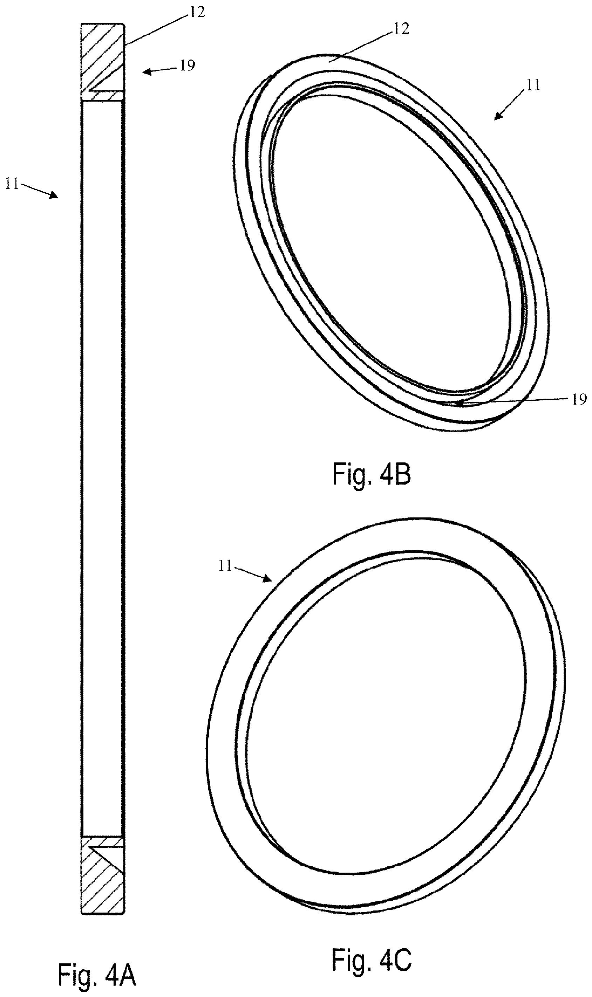

Fig. 4A zeigt das Folienhalteelement 11 aus Fig. 1 isoliert in Schnittansicht. Das Folienhalteelement 11 weist vorzugsweise eine umlaufende Nut 19 auf, wobei die Nut 19 bevorzugt in einer Folienkontaktierungsfläche 12 V-förmig ausgebildet ist. Das Folienhalteelement 11 ist ein von dem Kopplungselement 1 gesondertes Teil. Deswegen kann das Folienhalteelement 11 aus einem anderen Material als das Kopplungselement 1 ausgebildet sein, wodurch der Vorteil erhalten wird, dass sich das Folienhalteelement 11 aus einem Material, welches besonders gut an einer Folie 10 haftet, bereitgestellt werden kann, ohne dass das gesamte Kopplungselement 1 aus einem derartigen Material bereitgestellt werden muss. Das Folienhalteelement 11 kann dabei bevorzugt aus einem Kunststoff bestehen. Das Folienhalteelement 11 kann aber auch aus dem gleichen Material wie das Kopplungselement 1 bereitgestellt werden, wobei die Folienkontaktierungsfläche 12 eine andere Oberfläche bereitstellen kann. Beispielsweise kann die Folienkontaktierungsfläche 12 aufgeraut sein, sodass die Folie 10 besser daran haften kann an als an einem, beispielsweise, glattem polierten Kopplungselement 1.

Es soll besonders hervorgehoben werden, dass das Folienhalteelement 11, im Gegensatz zur Orientierung des Folienhalteelements 11 der Fig.1, auch verkehrt in der Folienelementvertiefung 16 ausgebildet werden kann, sodass die Folie 10 plan an der ersten Anschlussebene 7 anliegt, wobei die umlaufende Nut 19 in einer der Folienkontaktierungsfläche 12 abgewandten Fläche ausgebildet ist. Es soll dabei erwähnt sein, dass die Kopplungselemente 1 der Fig.1 derartig ausgebildet sein können, dass das linke Kopplungselement 1, ein verkehrt eingebrachtes Folienhalteelement 16 aufweisen kann, im Gegensatz dazu wie es in Fig. 1 dargestellt ist. Das rechte Kopplungselement 1 kann dabei ein wie in Fig. 1 dargestellt orientiertes Folienhalteelement 11 aufweisen. Mit anderen Worten kann das Folienhalteelement 11 in beliebiger Orientierung in der Folienelementvertiefung 16 verbracht sein.

Fig. 4B zeigt das Folienhalteelement 11 aus Fig. 4A in einer ersten isometrischen Ansicht, sodass dessen Nut 19 zu erkennen ist. Fig. 4C zeigt hingegen das Folienhalteelement 11 aus Fig. 4A in einer zweiten isometrischen Ansicht, sodass dessen flache Rückseite zu erkennen ist.

Fig. 5 zeigt die Flanschverbindung 21 aus Fig. 1 in einer isometrischen Ansicht. Dabei ist hervorgehoben, dass das Verbindungselement 22 und das weitere Verbindungselement 32 vorzugsweise als Überwurfklemme ausgebildet sein können. Diese Ausgestaltung des Verbindungselements 22 stellt den Vorteil bereit, dass eine hohe Vorspannkraft bei der Aufnahme des ersten Kopplungselements 1 und des anderen Kopplungselements 1 im Bereich der Öffnungsenden 3, 4 bereitgestellt werden kann. Hierdurch wird die mechanische Stabilität der Flanschverbindung 21 erhöht, und das Risiko einer Kontamination beim Abziehen der Folien 10 reduziert. Die Ausgestaltung als Überwurfklemme ermöglicht außerdem ein einfaches Anbringen und Abnehmen des Verbindungselements 22 und sorgt für einen hohen Anpressdruck beim Koppeln.

Das gesonderte Verbindungselement 22 ist dazu ausgebildet, das Kopplungselement 1 und das andere Kopplungselement 1 im Bereich der kegelstumpfförmigen Aufweitung 20 abschnittsweise aufzunehmen. Im Zuge der Aufnahme der Kopplungselemente 1 durch das Verbindungselement 22 werden die mit den Folien 10 (Folien 10 nicht dargestellt) verschlossenen Öffnungsenden 3 aneinandergepresst. Das Verbindungselement 22 weist eine, im Wesentlichen in der Anschlussebene 7 angeordnete Ausnehmung 23 zur Durchführung der Folien durch das Verbindungselement 22 auf. Die Ausnehmung 23 ist vorzugsweise schlitzförmig ausgebildet. Die Ausnehmung 23 ermöglicht die Einhaltung eines immer gleichen Abziehwinkels der Folien 10 durch einen Benutzer. Das Verbindungselement 22, 32 ist vorzugsweise aus Stahl gefertigt.

Es sei angemerkt, dass die Kopplungselemente 1 vorzugsweise miteinander koppelbare Lokalisationselemente 27 aufweisen, welche in Fig. 2A bis Fig. 3B ersichtlich sind. Die Lokalisationselemente 27 sind dazu ausgebildet, im Zuge einer Kopplung die ersten Öffnungsenden 3 im Wesentlichen deckungsgleich zueinander zu positionieren. Hierdurch wird eine optimale Positionierung des Kopplungselements 1 und des anderen Kopplungselements 1 sichergestellt. Vorzugsweise sind die Lokalisationselemente 27, als Nuten und Stifte ausgebildet, die jeweils ineinander eingreifen.

Angemerkt soll sein, dass obwohl in allen Figuren ausschließlich zylinderförmige Kopplungselemente 1 mit einem kreisförmigen Querschnitt dargestellt werden, auch andere Querschnittsformen bereitgestellt werden können. Beispielsweise soll ein rechteckiger Querschnitt genannt sein.

Ist sei außerdem angemerkt, dass in einer Flanschverbindung 21 auch Kopplungselemente 1 unterschiedlicher Ausführungsform vorliegen können. So kann lediglich eines der beiden Kopplungselemente 1 ein Dichtungselement 13 aufweisen, oder die Folienhalteelemente 11 und oder Dichtungselemente 13 auf unterschiedlicher Höhe ausgebildet sein.

Auch soll angemerkt sein, dass das Kopplungselement 1 auch über zusätzliche Durchgangslöcher aufweisen kann, mit welchen eine Verbindung hergestellt werden kann. Darunter fallen beispielsweise im Flanschteil angeordnete Bohrungen, durch welche Schrauben, als Verbindungselement 22, durchführbar sind, um mit einem anderen Kopplungselement 1 verbunden zu werden.

Des Weiteren sei angemerkt, dass auch mehrere voneinander getrennte, bzw. nebeneinander liegende Dichtungselemente 13, und/oder Folienhalteelemente 11 in einem Kopplungselement 1 vorliegen können. So kann beispielsweise ein Folienhalteelement 11 nicht im Bereich einer Anschlussebene 14 liegen und ein anderes im Bereich der Anschlussebene 14 liegen. Beide Folienhalteelemente 1 können mit einer einzelnen Folie 10 verbunden sein, oder eine zweite Folie 10 kann mit dem Folienhalteelement 1, welches nicht im Bereich der Anschlussebene 13 liegt, verbunden sein. In beiden Fällen ist ein besonders steriles Kopplungselement 1 erhalten, da ein größerer Bereich von einer Folie 10 verdeckt ist. Mit anderen Worten kann die Folie 10 bzw. die zweite Folie in zweiter Funktion als eine Art Verpackung, die mit dem Kopplungselement 1 verbunden ist, für das Kopplungselement 1 angesehen werden.

Es sei abschließend angemerkt, dass einer Fachperson klar ist, dass das erfindungsgemäße Folienhalteelement 1 auch am zweiten Öffnungsende 4, gleich dem Folienhalteelement 1 des ersten Öffnungsendes 3 bereitgestellt werden kann. Dadurch ist der Vorteil erhalten, dass beide Öffnungsenden 3, 4 steril gekoppelt werden können.

Die fluidführende Fläche 2 kann beispielsweise elongierter sein, als in den Figuren darstellt ist. Das gleiche gilt, beispielsweise, für die kegelstumpfförmigen Aufweitungen 20, 30, welche mit einem anderen Winkel als dargestellt bereitgestellt werden können.

## Patentansprüche

1. Kopplungselement (1) für eine sterile Flanschverbindung (21) zur Verbindung von Fluidkanälen, umfassend:
- eine fluidkontaktierende Fläche (2),
- ein erstes Öffnungsende (3) und ein zweites Öffnungsende (4) der fluidkontaktierenden Fläche (2),
- einen Flanschteil (5) am ersten Öffnungsende (3) und/ oder am zweiten Öffnungsende (4),
- eine Anschlussebene (7, 8), wobei die Anschlussebene (7, 8) normal zu einer axialen Kopplungselementachse (9) des Kopplungselements (1) angeordnet ist, und das jeweilige Öffnungsende (3, 4) des Kopplungselements (1) begrenzend abschließt,
- und eine Folie (10), wobei die Folie (10) zumindest das erste Öffnungsende (3) der fluidkontaktierenden Fläche (2) verschließt, wobei das Kopplungselement (1) zumindest ein von dem Kopplungselement (1) gesondertes Folienhalteelement (11) mit einer Folienkontaktierungsfläche (12), an der die Folie (10) abziehbar verbunden ist, umfasst,
**dadurch gekennzeichnet, dass**
das zumindest eine Folienhalteelement (11) im Bereich (14) der Anschlussebene (7) ausgebildet ist und das zumindest ein Folienhalteelement (11) in zumindest einer Folienhalteelementvertiefung (16) ausgebildet ist, wobei die Folienkontaktierungsfläche (12) des Folienhalteelements (11) mit der Anschlussebene (7) abschließt, wobei die Folienhalteelementvertiefung (16) in den Flanschteil (5) eingearbeitet ist.

2. Kopplungselement (1) gemäß dem Anspruch 1, **dadurch gekennzeichnet, dass** zumindest ein das erste Öffnungsende (3) umlaufendes Dichtungselement (13) im Bereich der Anschlussebene (14) ausgebildet ist.

3. Kopplungselement (1) gemäß dem Anspruch 2, **dadurch gekennzeichnet, dass** das zumindest eine Dichtungselement (13) in zumindest einer Dichtungselementvertiefung (15) ausgebildet ist, wobei das Dichtungselement (13) über die Anschlussebene (7) hinausgehend ist und die Dichtungselementvertiefung (15) in den Flanschteil (5) eingearbeitet ist.

4. Kopplungselement (1) nach Anspruch 2, **dadurch gekennzeichnet, dass** ein Umfang des Folienhalteelements (11) größer als ein Umfang des Dichtungselements (13) ist.

5. Kopplungselement (1) nach einem der Ansprüche 1 bis 4, **dadurch gekennzeichnet, dass** das Folienhaltelement (11) aus Kunststoff, bevorzugt aus Polyethylen, PE 1000 REIN, PPSU, PES, PSU, PVDF, PET, POM-H, POM-C, PA 6 G 210, PA 66, PA 6, PP, PE-UHMW, PE 500, PE 300, oder PE 100, ausgebildet ist, und/oder, dass das Kopplungselement (1) aus Metall, bevorzugt aus Stahl, besonders aus poliertem Stahl, ausgebildet ist.

6. Kopplungselement (1) nach einem der Ansprüche 1 bis 5, **dadurch gekennzeichnet, dass** zumindest ein Durchgangsloch (17) im Flanschteil (5) ausgebildet ist, wobei das Folienhaltelement (11) durch das Durchgangsloch (17) erreichbar ist.

7. Kopplungselement (1) nach einem der Ansprüche 1 bis 6, **dadurch gekennzeichnet, dass** die Folienhalteelementvertiefung (16) Widerhaken (18) ausbildet.

8. Kopplungselement (1) nach einem der Ansprüche 1 bis 7, **dadurch gekennzeichnet, dass** das Folienhalteelement (11) zumindest eine umlaufende Nut (19) aufweist, wobei die Nut (19) bevorzugt in der Folienkontaktierungsfläche (12), und/oder in einer der Folienkontaktierungsfläche (12) angrenzenden Fläche und/oder in einer der Folienkontaktierungsfläche (12) abgewandten Fläche V-förmig ausgebildet ist.

9. Kopplungselement (1) nach einem der Ansprüche 1 bis 8, **dadurch gekennzeichnet, dass** im Bereich des Öffnungsendes (3, 4) eine kegelstumpfförmige Aufweitung (20, 30) ausgebildet ist.

10. Flanschverbindung (21), umfassend zwei Kopplungselemente (1) nach einem der Ansprüche 1 bis 9 und ein Verbindungselement (22) zum lösbaren Verbinden der zwei Kopplungselemente (1).

## Claims

1. Coupling member (1) for a sterile flange connection (21) for connecting fluid channels, comprising:
- a fluid-contacting surface (2),
- a first opening end (3) and a second opening end (4) of the fluid-contacting surface (2),
- a flange part (5) at the first opening end (3) and/or at the second opening end (4),
- a connection plane (7, 8), wherein the connection plane (7, 8) is arranged perpendicular to an axial coupling element axis (9) of the coupling member (1) and terminates the respective opening end (3, 4) of the coupling element (1),
- and a foil (10), wherein the foil (10) closes at least the first opening end (3) of the fluid-contacting surface (2), wherein the coupling member (1) comprises at least one foil holding element (11) separate from the coupling member (1) with a foil contacting surface (12) to which the foil (10) is peelably connected,
**characterized in that**
the at least one foil holding element (11) is formed in the region (14) of the connection plane (7) and the at least one foil holding element (11) is formed in at least one foil holding element recess (16), wherein the foil contacting surface (12) of the foil holding element (11) terminates with the connection plane (7), wherein the foil holding element recess (16) is incorporated in the flange part (5).

2. Coupling member (1) according to claim 1, **characterized in that** at least one sealing element (13) surrounding the first opening end (3) is formed in the region of the connection plane (14).

3. Coupling member (1) according to claim 2, **characterized in that** the at least one sealing element (13) is formed in at least one sealing element recess (15), wherein the sealing element (13) is extending beyond the connection plane (7) and the sealing element recess (15) is incorporated into the flange part (5).

4. Coupling member (1) according to claim 2, **characterized in that** a circumference of the foil holding element (11) is larger than a circumference of the sealing element (13).

5. Coupling member (1) according to any one of claims 1 to 4, **characterized in that** the foil holding element (11) is made of plastic, preferably of polyethylene, PE 1000 REIN, PPSU, PES, PSU, PVDF, PET, POM-H, POM-C, PA 6 G 210, PA 66, PA 6, PP, PE-UHMW, PE 500, PE 300, or PE 100, and/or **in that** the coupling element (1) is made of metal, preferably of steel, particularly of polished steel.

6. Coupling member (1) according to any one of claims 1 to 5, **characterized in that** at least one through-hole (17) is formed in the flange part (5), wherein the foil holding element (11) is accessible through the through-hole (17).

7. Coupling member (1) according to any one of claims 1 to 6, **characterized in that** the foil holding element recess (16) forms barbs (18).

8. Coupling member (1) according to any one of claims 1 to 7, **characterized in that** the foil holding element (11) has at least one circumferential groove (19), wherein preferably the groove (19) is V-shaped in the foil contacting surface (12) and/or in a surface adjacent to the foil contacting surface (12) and/or in a surface facing away from the foil contacting surface (12).

9. Coupling member (1) according to any one of claims 1 to 8, **characterized in that** a frustoconical expansion (20, 30) is formed in the region of the opening end (3, 4).

10. Flange connection (21), comprising two coupling member (1) according to any one of claims 1 to 9 and a connecting element (22) for detachably connecting the two coupling member (1).

## Revendications

1. Élément de couplage (1) pour un raccord à bride stérile (21) destiné à relier des canaux de fluide, comprenant:
- une surface en contact avec le fluide (2),
- une première extrémité d'ouverture (3) et une deuxième extrémité d'ouverture (4) de la surface en contact avec le fluide (2),
- une partie de bride (5) à la première extrémité d'ouverture (3) et/ou à la deuxième extrémité d'ouverture (4),
- un plan de connexion (7, 8), le plan de connexion (7, 8) étant disposé perpendiculairement à un axe d'élément de couplage axial (9) de l'élément de couplage (1) et fermant l'extrémité d'ouverture respective (3, 4) de l'élément de couplage (1) de manière limitative,
- et un film (10), le film (10) fermant au moins la première extrémité d'ouverture (3) de la surface en contact avec le fluide (2), l'élément de couplage (1) comprenant au moins un élément de maintien de film (11) séparé de l'élément de couplage (1) et ayant une surface de contact avec le film (12) à laquelle le film (10) est relié de manière amovible,
**caractérisé en ce que** l'au moins un élément de maintien de film (11) est formé dans la région (14) du plan de connexion (7) et l'au moins un élément de maintien de film (11) est formé dans au moins un évidement de l'élément de maintien de film (16), la surface de contact avec le film (12) de l'élément de maintien de film (11) se terminant par le plan de connexion (7), l'évidement de l'élément de maintien de film (16) étant intégré dans la partie de bride (5).

2. Elément de couplage (1) selon la revendication 1, **caractérisé en ce qu'**au moins un élément d'étanchéité (13) entourant la première extrémité d'ouverture (3) est formé dans la région du plan de connexion (14).

3. Elément de couplage (1) selon la revendication 2, **caractérisé en ce que** l'au moins un élément d'étanchéité (13) est formé dans au moins un évidement de l'élément d'étanchéité (15), l'élément d'étanchéité (13) s'étendant au-delà du plan de connexion (7) et l'évidement de l'élément d'étanchéité (15) étant intégré dans la partie de bride (5).

4. Elément de couplage (1) selon la revendication 2, **caractérisé en ce qu'**une circonférence de l'élément de maintien de film (11) est plus grande qu'une circonférence de l'élément d'étanchéité (13).

5. Elément de couplage (1) selon l'une des revendications 1 à 4, **caractérisé en ce que** l'élément de maintien de film (11) est formé de matière plastique, de préférence de polyéthylène, PE 1000 PUR, PPSU, PES, PSU, PVDF, PET, POM-H, POM-C, PA 6 G 210, PA 66, PA 6, PP, PE-UHMW, PE 500, PE 300 ou PE 100, et/ou **en ce que** l'élément de couplage (1) est formé de métal, de préférence de acier, en particulier de acier poli.

6. Elément de couplage (1) selon l'une des revendications 1 à 5, **caractérisé en ce qu'**au moins un trou traversant (17) est formé dans la partie de bride (5), l'élément de maintien de film (11) étant accessible à travers le trou traversant (17).

7. Elément de couplage (1) selon l'une des revendications 1 à 6, **caractérisé en ce que** l'évidement de l'élément de maintien de film (16) forme des barbes (18).

8. Elément de couplage (1) selon l'une des revendications 1 à 7, **caractérisé en ce que** l'élément de maintien de film (11) présente au moins une rainure périphérique (19), la rainure (19) étant, de préférence, en forme de V dans la surface de contact avec le film (12) et/ou dans une surface adjacente à la surface de contact avec le film (12) et/ou dans une surface opposée à la surface de contact avec le film (12).

9. Elément de couplage (1) selon l'une des revendications 1 à 8, **caractérisé en ce qu'**un élargissement tronconique (20, 30) est formé dans la région de l'extrémité d'ouverture (3, 4).

10. Raccord à bride (21) comprenant deux éléments de couplage (1) selon l'une des revendications 1 à 9 et un élément de liaison (22) pour relier de manière détachable les deux éléments de couplage (1).
